# DEMANDE DE BREVET EUROPEEN

(11) **EP 0 688 537 A1**
(43) Date de publication de la demande: **27.12.1995**
(21) Numéro de dépôt: 95401436.1
(22) Date de dépôt: 19.06.1995
(51) Int. Cl.: A61B 17/54, A61B 17/32

(54) **Appareil pour extraire les cors au pied**

(30) Priorité: 20.06.1994 FR 9407521
(71) Demandeur: PODIAFRANCE (S.a.r.l.), F-92380 Garches (FR)
(72) Inventeur: Alcaraz, René-Gérard, 92380 Garches (FR); Minel, Dominique, 52330 Montheries (FR)
(74) Mandataire: Petit, Hélène

(57) **Abrégé**

Appareil pour extraire les cors au pied, comportant une lame (9) avec une extrémité coupante (10), profilée en forme de gouge hémicylindrique, et un corps de lame (11) se prolongeant à l'opposé de son extrémité coupante par une partie plate (14), la lame étant adaptée à s'engager dans une fente étroite (8) ménagée entre deux languettes souples (8a, 8b) prévues en bout d'un manche de manipulation (2), muni d'une bague ou analogue (12), coopérant avec un filetage (4) ménagé sur le manche pour serrer les deux languettes l'une vers l'autre en immobilisant le corps de lame dans la fente, au-delà de laquelle dépasse l'extrémité coupante, caractérisé en ce que le corps de lame comporte, en partie au moins dans la fente du manche, une nervure en relief (16) propre à rigidifier la lame.

## Description

La présente invention est relative à un instrument de podologie et concerne plus particulièrement un appareil pour extraire les cors au pied d'un patient.

On connaît déjà dans la technique des instruments de ce genre qui, de façon générale, sont constitués par une lame coupante dont l'extrémité est profilée avec une partie creuse formant une sorte de canal hémicylindrique à la façon d'une gouge, de manière à permettre une action de coupe par son bord effilé et de creusement de la peau sous le cor à extraire, cette lame étant solidaire de l'extrémité d'un manche formant avec elle un ensemble monobloc.

Cette solution présente toutefois des inconvénients du fait de l'usure du tranchant de la lame qu'il convient donc d'affûter périodiquement et en tous cas de stériliser avec le manche entre chaque usage, ce qui n'est pas pratique et entraîne une perte de temps, voire un coût notable si l'affûtage doit être réalisé par un spécialiste qui n'est pas podologue lui-même.

Une autre solution connue consiste à utiliser un manche muni d'une tête adaptée à recevoir une lame séparable, pouvant ainsi être changée après chaque usage. Dans ce cas, la lame comporte une partie postérieure profilée en forme de gouge comme dans la première variante et une extrémité antérieure opposée, généralement plane, propre à s' enfiler dans une fente axiale, ménagée longitudinalement en bout du manche et délimitée entre deux languettes solidaires de celui-ci formant pince, ces languettes étant forcées l'une contre l'autre pour serrer la lame et l'immobiliser vis-à-vis du manche au moyen d'une bague vissée contre un filetage prévu sur ce dernier dans une partie conique ménagée au droit de la fente afin de rapprocher les deux languettes.

Un tel instrument évite pour partie les inconvénients de la solution classique ; toutefois, il se révèle encore peu satisfaisant, notamment du fait que la lame portée par le manche et faisant largement saillie hors de celui-ci en le prolongeant à son extrémité n'est pas suffisamment rigide pour assurer la précision du geste exigée par son usage. En effet, cette lame interchangeable est généralement très fine et étroite, donc flexible, de telle sorte que sa manipulation lors de l'extraction d'un cor est délicate, voire mal adaptée.

La présente invention est relative à un appareil qui pallie ces défauts en conférant à la lame une rigidité accrue lors de son montage dans un manche, conformément à la seconde solution rappelée ci-dessus, en combinant ainsi tous les avantages de cette dernière sans entraîner ses inconvénients.

A cet effet, l'appareil considéré, comportant une lame avec une extrémité coupante, profilée en forme de gouge hémicylindrique et un corps de lame présentant une nervure en relief propre à rigidifier cette lame, qui se prolonge à l'opposé de son extrémité coupante par une partie plate, la lame étant adaptée à s'engager dans une fente étroite ménagée entre deux languettes souples prévues en bout d'un manche de manipulation, muni d'une bague ou analogue, coopérant avec un filetage ménagé sur le manche pour serrer les deux languettes l'une vers l'autre en immobilisant le corps de lame dans la fente, au-delà de laquelle dépasse l'extrémité coupante, se caractérise en ce que la nervure s'engage au moins en partie dans la fente du manche.

Selon une caractéristique particulière, l'une des languettes du manche dans la fente comporte une rainure longitudinale, apte à accommoder la nervure en relief de manière à solidariser étroitement la lame et le manche. Avantageusement, la partie plate de la lame comporte par ailleurs un évidement central, facilitant sa tenue lors de son affûtage une fois séparée du manche.

Selon une autre caractéristique également, la nervure en relief présente une section en U ou en V. Enfin et selon des dispositions en elles-mêmes connues, la lame présente à son extrémité coupante un contour arrondi et éventuellement une largeur variable depuis cette extrémité.

D'autres caractéristiques d'un appareil pour extraire les cors au pied, établi conformément à l'invention, apparaîtront encore à travers la description qui suit d'un exemple de réalisation, donné à titre indicatif et non limitatif, en référence au dessin annexé sur lequel
- La Figure 1 est une vue en élévation et en coupe longitudinale partielle d'un appareil d'extraction de cors, connu dans la technique.
- La Figure 2 est une vue de détail à plus grande échelle de l'extrémité de l'appareil selon la Figure 1, avec une partie coupée pour faire apparaître la fente de montage de la lame coupante.
- La Figure 3 est une vue analogue à la Figure 2 mais relative à un appareil perfectionné conformément à l'invention.
- La Figure 4 est une vue en coupe longitudinale de l'extrémité de l'appareil selon la Figure 3.
- Les Figures 5 et 6 sont des vues en coupe transversale de la Figure 4, respectivement selon les lignes V-V et VI-VI de cette dernière.

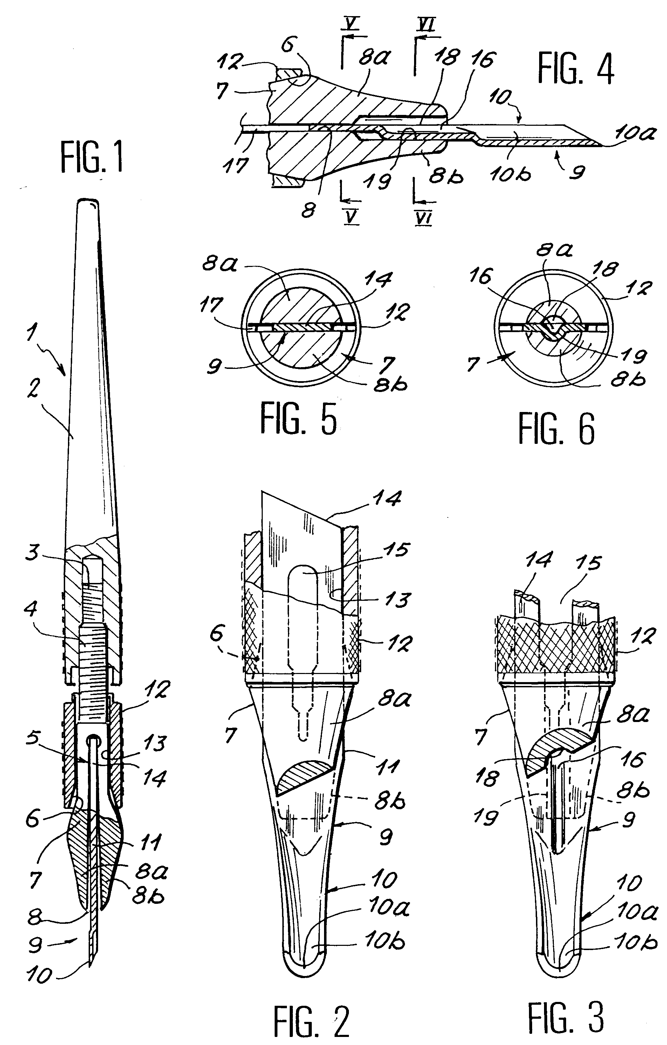

Sur la Figure 1, la référence 1 désigne un appareil d'extraction de cors classique dans la technique correspondante. Cet appareil se compose essentiellement d'un manche allongé 2, dont le profil est déterminé pour permettre une saisie appropriée de l'appareil par la main de l'intervenant, tenant le manche à la manière d'un porte-plume.

Le manche 2 comporte à son extrémité un logement borgne 3, taraudé intérieurement et dans lequel est susceptible de se visser un embout 4 prolongeant une tête amovible 5. Cette dernière présente, à l'opposé du manche 2, un profil formé de deux parties sensiblement coniques, respectivement 6 et 7, dans le prolongement l'une de l'autre et accolées par leurs bases.

La tête 5 comporte, dans l'axe longitudinal du manche 2, une fente étroite 8 délimitée par deux languettes respectivement 8a et 8b se faisant face à face et dans laquelle peut s'engager une lame amovible 9.

La lame 9 présente une extrémité coupante 10, comportant en bout une partie affûtée 10a, de profil arrondi comme représenté sur la Figure 2, et une section hémicylindrique dans sa partie courante 10b, de manière à la conformer sensiblement comme une gouge, ces dispositions en elles-mêmes connues permettant à la lame à la fois de couper la peau par sa partie 10a et de creuser sous le cor à extraire selon la technique habituelle pour ce genre d'intervention. La partie courante 10b va de préférence en s'évasant à partir de l'extrémitée affûtée 10a.

La partie centrale ou corps 11 de la lame 9, monté dans la fente 8 de la tête 5 est immobilisé par serrage mutuel des languettes 8a et 8b grâce à une bague creuse 12, comportant un filetage interne 13 coopérant avec celui de l'embout 4, de telle sorte que l'extrémité de cette bague dirigée vers la lame vienne progressivement s'appuyer contre la partie conique 6 de la tête 5 en forçant progressivement les deux languettes l'une vers l'autre, l'extrémité coupante 10 débordant à l'extrémité du manche sur une distance suffisante.

La lame 9 comporte enfin, à l'opposé de son extrémité coupante 10, une partie plane 14 comportant un évidement central 15 permettant d'immobiliser la lame lorsque cette dernière, extraite du manche 2, est placée dans une machine d'affûtage (non représentée).

L'expérience montre qu'une telle lame dont le caractère amovible vis-à-vis du manche de l'appareil présente certains avantages déjà mentionnés, notamment du fait qu'elle évite d'avoir à stériliser l'ensemble après chaque usage, seule la lame étant à changer, l'affûtage de celle-ci étant en outre facilité, comporte encore un défaut qui se révèle particulièrement préjudiciable.

En effet, une telle lame amovible n'est pas suffisamment rigide pour répondre à l'exigence essentielle réclamée par son usage, dans lequel les gestes de l'intervenant doivent être extrêmement précis et accomplis avec la plus grande sûreté pour éviter de blesser la chair entourant le cor à extraire.

L'invention consiste alors à prévoir sur le corps 11 de la lame, entre son extrémité coupante 10 conformée en gouge et sa partie plate opposée 14 maintenue dans le fond de la fente 8 du manche, une nervure en relief 16 s'étendant au moins en partie à l'intérieur de la fente, cette nervure présentant une longueur adaptée à conférer à la lame une rigidité appropriée qui pallie l'inconvénient ci-dessus tout en conservant les avantages de la solution classique et en particulier le caractère amovible de la lame.

A cet effet et comme représenté sur les Figures 3 à 6, on aménage le manche 2 de manière à ce que la fente 8 présente deux parties successives, respectivement 17 et 18, la première située au fond de la fente étant étroite et présentant une largeur suffisante pour accommoder la partie d'extrémité plate 14 de la lame, tandis que la seconde partie 18 est sensiblement plus large, de manière à permettre l'engagement dans celle-ci de la nervure 16, qui vient en particulier se loger dans une rainure 19 de la languette correspondante de la tête 5, en l'espèce la languette 8b sur les Figures 4 et 6.

La nervure 16 peut présenter une section droite différente d'une réalisation à l'autre, de préférence néanmoins en forme de U ou de V. Cette nervure présente une hauteur de l'ordre de quelques dizièmes de mm, de préférence de 2 dizièmes, pour une longueur approximativement voisine de 1 cm, la lame ayant dans sa partie courante une épaisseur généralement égale à 4 dizièmes. Par ailleurs, l'extrémité en forme de gouge hémicylindrique 10b de l'extrémité coupante de la lame peut présenter une largeur variable selon les différents modèles de lames utilisés.

On réalise ainsi un appareil d'extraction de cors à lame amovible, qui présente une rigidité très sensiblement améliorée, ce qui facilite considérablement la précision de son utilisation, en procurant de ce fait une amélioration remarquable vis-à-vis des solutions classiques.

La lame fixée sur le manche est de préférence jetable après un usage unique, cette lame étant fournie sous un emballage stérile de manière à éviter toute contamination ultérieure. Toutefois et en variante éventuelle, au moins dans certaines circonstances, cette lame peut être utilisée plusieurs fois, notamment après un affûtage approprié. Par ailleurs, il va de soi que l'invention ne se limite pas à l'exemple de réalisation plus spécialement décrit ci-dessus en référence au dessin annexé ; elle en embrasse au contraire toutes les variantes.

## Revendications

**1 -** Appareil pour extraire les cors au pied, comportant une lame (9) avec une extrémité coupante (10), profilée en forme de gouge hémicylindrique et un corps de lame (11) présentant une nervure en relief (16) propre à rigidifier cette lame, qui se prolonge à l'opposé de son extrémité coupante par une partie plate (14), la lame étant adaptée à s'engager dans une fente étroite (8) ménagée entre deux languettes souples (8a, 8b) prévues en bout d'un manche de manipulation (2), muni d'une bague ou analogue (12), coopérant avec un filetage (4) ménagé sur le manche pour serrer les deux languettes l'une vers l'autre en immobilisant le corps de lame dans la fente, au-delà de laquelle dépasse l'extrémité coupante, caractérisé en ce que la nervure (16) s'engage au moins en partie dans la fente du manche.

**2 -** Appareil selon la revendication 1, caractérisé en ce que l'une des languettes (8a ou 8b) du manche (2) dans la fente (8) comporte une rainure longitudinale (19), apte à accommoder la nervure en relief (16) de manière à solidariser étroitement la lame (9) et le manche.

**3 -** Appareil selon l'une des revendications 1 ou 2, caractérisé en ce que la partie plate (14)de la lame (9) comporte un évidement central (15), facilitant sa tenue lors de son affûtage une fois séparée du manche.

**4 -** Appareil selon l'une quelconque des revendications 1 à 3, caractérisé en ce que la nervure en relief (16) présente une section en U ou en V.

**5 -** Appareil selon l'une quelconque des revendications 1 à 4, caractérisé en ce que la lame (9) présente à son extrémité coupante (10) un contour arrondi (10a) et éventuellement une largeur variable depuis cette extrémité.
